# EUROPEAN PATENT APPLICATION

(11) **EP 4 088 738 A1**
(43) Date of publication of application: **16.11.2022**
(21) Application number: 21173941.2
(22) Date of filing: 14.05.2021
(51) Int. Cl.: A61K 39/12, C07K 14/165

(54) **NOVEL PROTEIN AND NUCLEIC ACID SEQUENCES FOR COVID-19 VACCINES**

(71) Applicant: Dompé farmaceutici S.p.a., 20122 Milano (IT)
(72) Inventor: ALLEGRETTI, Marcello, 67100 L'Aquila (IT); CIMINI, Annamaria, 67019 L'Aquila (IT); BECCARI, Andrea Rosario, 80131 Napoli (IT); TALARICO, Carmine, 80131 Napoli (IT); MAURI, Elisabetta Maria Ester, 20122 Milano (IT)
(74) Representative: Mauri, Elisabetta Maria Ester

(57) **Abstract**

The present invention relates to a mutated SARS-CoV-2 spike protein, a variant or fragment thereof or an mRNA or DNA encoding them for use in the prevention of COVID-19.

## Description

### FIELD OF THE INVENTION

The present invention relates to proteins and nucleic acid sequences suitable for use in COVID-19 vaccines.

### STATE OF THE ART

Coronaviruses (Covs) are a large family of single-stranded, enveloped RNA viruses that belong to the Coronaviridae family. The limited number of coronaviruses known to be able to infect humans were considered in the past as relatively harmless respiratory human pathogens, causing mild infections. However, two coronavirus subtypes have emerged, Severe Acute Respiratory Syndrome Coronavirus (SARS-CoV) and the Middle East Respiratory Syndrome Coronavirus (MERS-CoV), which cause severe and sometimes fatal respiratory tract infections in humans (Pereira, H et al., 1989 Coronaviridae. Andrewes 'Viruses of Vertebrates, 5th ed.pp. 42-57, Holmes, K.V. et al., Virology 1996, 1: 1075-1093). In December 2019, atypical pneumonia cases occurred in China and the cause was later identified as being a novel coronavirus. The World Health Organization (WHO) named the virus as SARS-CoV-2 and the related disease as COVID-19.

The virus spread rapidly worldwide, and on 11 March 2020 the WHO declared SARS-CoV-2 infection as a pandemic. Most people infected with COVID-19 experience mild to moderate respiratory illness (fever, fatigue, dry cough and dyspnoea) and recover without requiring special treatments. Older people, and those with underlying medical problems like cardiovascular disease, diabetes, chronic respiratory disease, and cancer are more likely to develop serious illness. Furthermore, analysis of epidemiological and clinical characteristics and outcomes of patients infected by SARS-CoV-2 have shown that 15% of people with symptomatic COVID-19 have significant disease including severe pneumonia, and 5% experience critical disease with life-threatening complications. Critical disease includes acute respiratory distress syndrome (ARDS), sepsis, septic shock, cardiac disease, thromboembolic events, such as pulmonary embolism and multi-organ failure.

There is also growing evidence that in those who develop critical COVID-19 disease, long-term consequences such as rare neurological and psychiatric complications are reasonably expected. These may include stroke, delirium, anxiety, depression, damage or inflammation of the brain and sleep disturbances.

Due to the dramatic rise in cases and deaths worldwide and the social and economic consequences thereof, efforts have focused to the development of a safe and effective vaccines to help control and bring to an end the COVID-19 pandemic.

Different vaccines are under development and some have already been approved for prevention of COVID-19.

All COVID-19 vaccines and vaccine candidates act by inducing an immune response against virus protein antigens, making use of different strategies and technologies: traditional inactivated or live-attenuated virus vaccines, based on the introduction in the host of a number of different SARS-CoV-2 antigens, vaccines based on recombinantly produced SARS-CoV-2 proteins in association with adjuvants and gene-based vaccines, such as mRNA, DNA or vector vaccines, which deliver genes encoding the viral protein antigens for in vivo production in the cells of the host.

SARS-COV-2 is characterised by four structural proteins, namely spike (S) protein, envelope (E) protein, membrane (M) protein and nucleocapsid (N) protein (Chen Y et al, J Med Virol 2020, 92: 418-423).

Currently, most COVID-19 vaccines already approved or under development use the spike protein as target antigen. This protein is in fact considered an important antigenic determinant capable of inducing a protective immune response (Ou et al, Nat Commun 2020; 11: 1620). Furthermore, it is also an essential molecule for entry of the virus into cells through uses the cell entry receptor Angiotensin-converting enzyme II (ACE2). Therefore, antibodies targeting the S protein prevent the virus from entering and replicating inside the host cells.

The SARS-CoV-2 S protein has the 1273 aminoacid sequence of SEQ.ID. No: 1 and consists of a signal peptide located at the N-terminus (amino acids 1-13), the S1 subunit (14-685 residues) and the S2 subunit (686-1273 residues). The S1 subunit mediates receptor binding to ACE2 through the receptor binding domain (RBD) and the S2 subunit is responsible for the viral and host cell membrane fusion (Letko et al, Nat Microbiol 2020, 5: 562-569, Huang et al, Acta Pharmacologica Sinica 2020, 41:1141-1149). During the fusion process the S2 protein changes from a prefusion to a postfusion (hairpin) conformation. Subsequently, surface proteases cleave the S2 subunit (Silverira et al, Life Sci 2021, 267:1128919).

The vaccines approved so far by EMA are based on nucleic acids that induce the production in the host of the spike protein as protein antigen: the mRNA based Comirnaty vaccine developed by Pfizer and BioNTech, the mRNA based Moderna COVID-19 vaccine developed by Moderna and the DNA, the viral vector delivered DNA vaccines Vaxzevria developed by Oxford University and AstraZeneca and COVID-19 Vaccine Janssen by Janssen-Cilag International.

Other vaccines based on these approaches are under development as well as vaccines using recombinantly produced SARS-CoV-2 spike protein as the active ingredient, for example the vaccine NVX-CoV2373 by Novavax and Co-VLP by Medicago and GSK (Forni et al, Cell Death & Differentiation 2021, 28:626-639).

Recent evidence has suggested a potential activity of the SARS-CoV-2 spike protein on cell signalling that is independent from facilitation of viral infection.

In particular, it has been shown that the spike protein can induce pro-inflammatory responses *in vitro* in murine and human macrophages (Shirato et al, Heyon 2021, 7(2) e06187) and can activate cell signaling events in cultured human vascular cells (Suzuki et al, Vascular Pharmacology 2021, 137: 106823).

This aspect is very relevant since most of the COVID-19 vaccines introduce the spike protein in the organism.

It has been reported that after administration of the mRNA and DNA vaccines approved so far by intramuscular injection, the spike protein is expressed mainly in myocites, fibroblasts, dendritic cells and lymphocytes but levels of expression have been detected with some of the vaccines also in other organs (information available at https://www.ema.europa.eu/).

In view of the above, it therefore needs to be investigated whether the presence in the body of the spike protein produced by vaccination may interfere with biological processes, thereby causing side effects in the short and long term.

Nuclear hormone receptors are a family of ligand-regulated transcription factors that are activated by thyroid and steroid hormones and various other lipid-soluble signals, including retinoic acid and vitamin D.

Steroid hormones, in particular androgens and estrogens, have been shown to regulate a wide range of physiological functions such as development and maintenance of secondary sexual characteristics, metabolism, blood salt balance, immune and inflammatory responses, response to stress and/or neuronal function.

Furthermore, an unbalance in the level or activity of these hormones have been implicated in many pathological processes.

In particular, estrogens have been implicated in the etiology of breast, endometrial, renal, and uterine cancer (Okamoto et al, Toxicology Letters 2020, 318: 99-103), while a pivotal role of androgens has been demonstrated in the pathogenesis of prostate cancer (Cold Spring Harb Perspect Med 2017;7:a030452).

Furthermore, administration of exogenous androgens and estrogens have been associated to a disturbed regulation of numerous aspects of the hemostatic and fibrinolytic pathways that contribute to the generation of a prothrombotic milieu and to a higher risk of thrombotic events (R 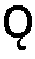 sca et al, J. Clin. Med. 2021, 10: 147; Abou- Ismail et al, Thrombosis Research 2020, 192: 40-51; Walker et al, JAMA Intern Med. 2020, 180(2):190-197).

The activity of these molecules is mediated by nuclear receptors, the estrogen and the androgen receptor, that function as hormone-activated transcription factors. These receptors are involved in diverse activities but share a remarkable structural and functional similarity. In the absence of the ligand they exist within target cells in a transcriptionally inactive form. Upon ligand activation, they bind to the promoters of target genes and form a transcriptional complex with a number of interacting proteins, collectively known as co-regulators, that can either activate (coactivators (CoA)) or inactivate (corepressors (CoR)) transcriptional activity, thereby triggering the activation or repression of target gene expression (Patel et al, Pharm Ther 2018, 186: 1-24; Zella et al, Arch Biochem Biophys 2007, 460(2): 206-212). The interaction between nuclear receptors and coactivators is mediated by LxxLL motifs (where L is leucine and x is any amino acid) present and shared within the coactivator proteins of the different nuclear hormone receptor. These motifs are necessary and sufficient for the binding of these proteins to the receptor and for enhancing its transcriptional activity (Patel et al, Pharm Ther 2018, 186: 1-24; Zella et al, Arch Biochem Biophys 2007, 460(2): 206-212; Chang et al, Trends Pharm Sci2005, 26(5): 225-228).

### SUMMARY OF THE INVENTION

The present inventors have now surprisingly found that the SARS-CoV-2 spike protein contains a number of LxxLL-like motifs homolog to that of the nuclear coactivator 1 (NCOA1), that are able to bind to the NCOA1 binding domain and activate the transcription activity of the estrogen and androgen receptors. Furthermore, the sequence homology of the NCOA1 binding domain throughout the different nuclear receptors supports also a potential ability of the spike protein to also activate transcription induced by other nuclear receptors, such as the Vitamin D and thyroid hormone receptor.

From this finding derives that the spike protein produced or contained in vaccines can interfere with the activity of nuclear receptors, in particular the estrogen and androgen receptors, and create an unbalance between physiological and pathological activities mediated by these receptors, thus generating unwanted, serious side effects.

The present inventors have identified mutations in the LxxLL motifs of SARS-CoV-2 spike protein that abolish the binding activity of the protein to the NCOA1 binding domain of the estrogen and androgen receptors.

These sequences are therefore useful for the development of safer vaccines against COVID-19.

Accordingly, an object of the invention is a mutated SARS-CoV-2 spike protein, wherein at least two of the aminoacids isoleucine or leucine at a position corresponding to aminoacid 818, 821, 822, 841, 861, 864 and 865 of the wild type SARS-Cov-2 protein sequence (SEQ ID NO: 1) are replaced by alanine, or a variant thereof.

A further object of the invention is a mutated SARS-CoV-2 spike protein according to the invention has the aminoacid sequence of SEQ ID NO: 2.

A further object of the invention is a variant of a mutated SARS-CoV-2 spike protein according to the invention.

A further object of the invention is an immunogenic fragment of a protein or variant according to the invention.

A further object of the invention is an mRNA encoding for the mutated SARS- CoV-protein, a variant or an immunogenic fragment according to the invention.

A further object of the invention is said mutated SARS-CoV-2 spike protein, variant or fragment, mRNA or DNA for use in the prevention of COVID-19 in a subject.

A further object of the invention is a vaccine comprising said mutated SARS-CoV-2 spike protein, variant or fragment, mRNA or DNA.

A further object of the invention is said vaccine for use in the prevention of COVID-19 in a subject.

### DESCRIPTION OF THE FIGURES

Figure 1 shows the Estrogen receptor (ER) complexed with nuclear receptor coactivator. The whole complex is reported in the cartoon; the ER monomers are in grey surface, while the NCOA segments are reported in black cartoon and lines.
Figure 2 shows the network of the most significant interactions of ER1 and ER2. Among the most reliable interacting proteins, the Nuclear receptor COActivators (NCOAs) directly bind the nuclear receptors and stimulate transcriptional activities.
Figure 3 shows the sequence alignment among LDX region in spike and other LDX cofactor-receptor domains.
Figure 4 shows the 3D best docking hypothesis for spike-ER blind docking. The proteins are reported in cartoon. The ER dimer is in white cartoon while the spike protein in reported in black surface.
Figure 5 shows the 3D best docking hypothesis for spike-ER motif-oriented docking. The proteins are reported in cartoon. The ER dimer is in white cartoon while the spike protein is reported in black surface.
Figure 6 shows cell proliferation, expressed as percentage of control, induced by treatment of MCF-7 breast cancer cells with vehicle (CTR), estradiol (ESTR), wild type spike protein (SPIKE) or combinations of the two, either alone or in the presence of Raloxifene, measured as described in Example 2.
Figure 7 shows cell proliferation, expressed as percentage of control, induced by treatment of MCF-7 breast cancer cells with vehicle (CTR), peptide 2, 5 or 7, wild type spike protein, wild type spike protein plus raloxifene or raloxifene alone, measured as described in Example 2.
Figure 8 shows cell proliferation, expressed as percentage of control, induced by treatment of LNCap prostate cancer cells with peptide 2, 5 or 7 or wild type spike protein, as described in Example 2.
Figure 9 shows results of proteomic analysis carried with Proteome Profiler Human XL Cytokine Array Kit (THP1 cell line differentiated with TPA/LPS and treated for 24h), after treatment with control, estrogen, wild type spike protein or wild type spike protein, as described in Example 3.

### DETAILED DESCRIPTION OF THE INVENTION

As will be described in details in the Experimental section, the present invention is based on the surprising finding that the SARS-CoV-2 spike protein exposes three LxxLL-like motifs that are able to mediate the binding and activation of nuclear receptor, in particular estrogen and androgen receptor. As will be shown in the Examples, each of these motifs individually are sufficient to activate proliferation of cancer cells induced by the estrogen or androgen receptor.

This finding raises concerns as regards potential side effects of COVID-19 vaccines that are based on the administration or the production in the host of the SARS-CoV-2 spike protein or of variants or fragments thereof that contain one or more of the above motifs

The inventors have found that the introduction of specific aminoacid mutations in correspondence with the three motifs identified abolishes the ability of the spike protein to interact with these receptors while not modifying the efficiency of the antigen-specific immune response against SARS-CoV-2.

Therefore, the use in new or commercially available spike based COVID-19 vaccines of a SARS-CoV-2 spike protein having a sequence carrying these mutations or an mRNA or DNA sequences encoding it, allows to maintain an equally effective immune response with a higher safety profile compared to the use wild type SARS-CoV-2 spike protein.

The present invention is directed to a mutated SARS-CoV-2 spike protein, variants and fragment thereof as well as mRNA and DNA encoding for the same, suitable to be used as active ingredient in vaccines for the prevention of COVID-19.

Accordinlgy, a first object of the invention is a mutated SARS-CoV-2 spike protein or variant thereof, wherein at least one of the aminoacids isoleucine or leucine at a position corresponding to aminoacid 818, 821, 822, 841, 861, 864 and 865 of the wild type SARS-Cov-2 protein sequence (SEQ ID NO: 1) are replaced by alanine.

Preferably, in said mutated SARS-CoV-2 spike protein according to the invention, at least the aminoacids 818, 821 and 822 or 841 or 861, 864 and 865 are replaced by alanine.

More preferably, all the above aminoacids are mutated to alanine.

Accordingly, preferably said mutated SARSCoV-2 spike protein according to the invention has the aminoacid sequence of SEQ ID NO: 2.

As discussed above, the aminoacid replacements introduced do not modify the conformation and antigenic properties of the SARS-CoV-2 spike protein.

A further object of the invention are variants of the mutated SARSCoV-2 spike protein described above.

According to the present invention, by "variant" of the mutated SARS-CoV-2 spike protein it is meant a protein having an aminoacid sequence which differs from the sequence of SEQ ID. NO: 2 in that it contains one or more substitutions, deletions or insertions of aminoacids. Preferably, said substitutions, deletions or insertions maintain the antigenic properties of the mutated SARS-CoV-2 spike protein according to the present invention. More preferably, said substitutions deletions or insertions improve the antigenic properties and/or stability of the mutated SARS-CoV-2 spike protein. Preferably, the variant has a sequence with an aminoacid identity with SEQ ID. NO: 2 of at least 90%, 95%, 98%, 99%.

Preferably, the variants according to the invention contain further aminoacid replacements in addition to the above described aminoacid replacements at positions 818, 821, 822, 841, 861, 864 and 865 of the wild type SARS-Cov-2 spike protein sequence.

In one preferred embodiment, said variants contain aminoacid replacements that stabilize the protein in the prefusion conformation. The use as an antigen of the spike protein in this conformation induces the production of antibodies that are able to respond and inactivate the virus in the early stages of the infection and before this enters into the cells, thus significantly increasing vaccine efficacy.

A number spike variants with aminoacid replacements that stabilize the spike protein in the prefusion state have already been identified. Among these, for example, is the mutant SARS-2-S spike protein (S-2P) having the aminoacid replacements K986P and V987P, which is encoded by the mRNA vaccine from both Pfizer/BioNTech (BNT162b) and Moderna (mRNA-1273). Furthermore, alternative variants of SARS-CoV-2 spike protein with a stable prefusion conformation and a higher yield of expression compared to S-2P have also been described in the literature. These are characterized by the replacements A892P and A942P or F817P and A899P. Finally, the most promising variant as regards stability and expression described in the literature is the HexaPro variant that includes all 6 aminoacid replacements described above.

According to this embodiment, a preferred object of the invention are variants of the mutated SARS-CoV-2 protein as described above, wherein at least two of the aminoacids alanine at positions 892, 899 and 942 of SEQ ID NO: 2, phenylalanine at position 817 of SEQ ID NO: 2, lysine at position 986 of SEQ ID NO: 2 and valine at position 987 of SEQ ID NO: 2 are replaced by proline.

Particularly preferred variants of the mutated SARS-CoV-2 spike protein according to this embodiment of the invention that stabilize the prefusion conformation of the mutated protein are provided hereinbelow.

A first variant of the mutated SARS-CoV-2 protein according to the invention, wherein the aminoacid lysine at position 986 of SEQ ID NO: 2 and the valine at position 987 of SEQ ID NO: 2 are replaced by proline (SEQ.ID.NO: 3).

A second variant of the mutated SARS-CoV-2 protein according to the invention, wherein the alanines at positions 892 and 942 of SEQ ID NO: 2 are replaced by proline (SEQ.ID.NO: 4).

A third variant of the mutated SARS-CoV-2 protein according to the invention, wherein the aminoacids phenylalanine at position 817 of SEQ ID NO: 2 and alanine at position 899 of SEQ ID NO: 2 and are replaced by proline (SEQ.ID.NO: 5).

A fourth variant of the mutated SARS-CoV-2 protein according to the invention, wherein the aminoacid lysine at position 986 of SEQ ID NO: 2, valine at position 987 of SEQ ID NO: 2 and alanine at positions 892 and 942 of SEQ ID NO: 2 are replaced by proline (SEQ.ID.NO: 6).

A fifth variant of the mutated SARS-CoV-2 protein according to the invention, wherein the aminoacids phenylalanine at position 817 of SEQ ID NO: 2, alanine at positions 892, 899 and 942 of SEQ ID NO: 2, lysine at position 986 of SEQ ID NO: 2 and valine at position 987 of SEQ ID NO: 2 are replaced by proline (SEQ.ID.NO: 7).

A sixth variant of the mutated SARS-CoV-2 protein according to the invention, wherein the aminoacids phenylalanine at position 817 of SEQ ID NO: 2, alanine at positions 892, 899 and 942 of SEQ ID NO: 2 are replaced by proline (SEQ.ID.NO: 8).

When SARS-CoV-2 spike protein is used as active ingredient in the COVID-19 vaccine, it may be advantageous to use a variant that makes the protein resistant to proteases. The vaccine candidate NVX-CoV2373 contains as active ingredient a variant of the SARS-CoV-2 spike protein having the aminoacid replacements R682Q, R683Q, R685Q that does not interfere with antigenic properties of the protein and confers a significantly increased protease resistance.

According to this embodiment, a preferred object of the invention are variants of the mutated SARS-CoV-2 spike protein according, wherein the aminoacid arginine in position 682, 683 and 685 of SEQ ID NO: 2 have been replaced by glutamine.

Particularly preferred variants according to this embodiment of the invention are those having the aminoacid sequence of SEQ. ID. NO: 9 to SEQ. ID. NO: 15 (variants 7 to 13). A number of COVID -19 vaccine candidates are based on immunogenic fragments of the SARS-CoV-2 spike protein or mRNA or DNA encoding for them (Forni et al, Cell Death & Differentiation 2021, 28: 626-639).

As demonstrated in the experimental section, when these fragments contain even only one of the LxxLL motifs identified by the inventors in the wikld type spike protein, they retain the ability to activate nuclear receptors.

Therefore, in the context of this type of vaccines, the use of immunogenic fragments of the mutated SARS-CoV-2 spike protein or variants thereof according to the present invention is advantageous for the reasons already discussed above.

Accordingly, a further object of the present invention is an immunogenic fragment of the mutated SARS-CoV-2 spike protein or of a variant thereof as described above, said fragment comprising at least one sequence selected from the following aminoacid sequences:
- aminoacid sequence from position 818 to position 822 of SEQ ID NO: 2,
- aminoacid sequence from position 841 to position 845 of SEQ ID NO: 2, and
- aminoacid sequence from position 861 to position 865 of SEQ ID NO: 2.

Accordingly, a further object of the present invention is an immunogenic fragment of the mutated SARS-CoV-2 spike protein or a variant thereof as described above, said fragment comprising a sequence corresponding the aminoacid sequences of from aminoacid 818 to aminoacid 865 of SEQ ID NO: 2.

A "fragment" of the mutated SARS-CoV-2 spike protein or a variant thereof according to the present invention refers to proteins having an aminoacid sequence which correspond to an N-terminally and/or C-terminally truncated sequence of SEQ ID. NO: 2 or of a variant thereof.

The immunogenic fragments according to the invention contain epitopes able to trigger protective immune responses in the host.

Epitopes of wild type SARS-CoV-2 spike protein that have a reduced risk of triggering autoimmune response and stimulate the production of protective antibodies have been identified, corresponding to sequences 232-246, 233-247, 471-503, 604-625, 817-833, 891-907, 897-913, 1164-1191, 1182-1209. On the contrary it was found that the peptide 597-603 indeces antibodies that enhance infection through a epitope sequence-dependent.

Accordingly, preferably, the immunogenic fragments according to the invention contain at least two, non-overlapping sequences selected from the following aminoacid sequences:
- aminoacid sequence from position 232 to position 246 of SEQ ID NO: 2,
- aminoacid sequence from position 233 to position 247 of SEQ ID NO: 2,
- aminoacid sequence from position 471 to position 503 of SEQ ID NO: 2,
- aminoacid sequence from position 604 to position 625 of SEQ ID NO: 2,
- aminoacid sequence from position 817 to position 833 of SEQ ID NO: 2,
- aminoacid sequence from position 891 to position 907 of SEQ ID NO: 2
- aminoacid sequence from position 897 to position 913 of SEQ ID NO: 2,
- aminoacid sequence from position 1164 to position 1191 of SEQ ID NO: 2, and
- aminoacid sequence from position 1182 to position 1209 of SEQ ID NO: 2

Preferably, the immunogenic fragment according to the invention comprises the aminoacid sequence from position 865 to position 1209 of SEQ ID NO: 2.

Preferably, the immunogenic fragment according to the invention comprises the aminoacid sequence from position 471 to position 817 of SEQ ID NO: 2.

Preferably, the immunogenic fragment according to the invention comprises the aminoacid sequence from position 232 to position 470 of SEQ ID NO: 2.

Preferably, the variants or fragments of the invention do not contain the aminoacid sequence from position 597 to position 603 of SEQ ID NO: 2.

A further object of the present invention are proteins comprising the aminoacid sequence of a mutated SARS-CoV-2 spike protein, variant or fragment thereof according to the present invention and one or more additional sequences.

Preferably, said additional sequences are not immunogenic and are used to confer the protein or fragment specific functional properties, for example as regards tissue distribution.

According to another preferred embodiment, said additional sequences are able to stimulate the immune response towards epitopes of the mutated SARS-CoV-2 spike protein, variant or fragment thereof.

A further object of the present invention is an mRNA encoding for the above described mutated SARS- CoV-2 spike protein, variant, peptide or protein.

According to a preferred embodiment, in order to increase stability and avoid in vivo degradation, said mRNA is a nucleoside modified mRNA sequence.

According to a preferred embodiment, in said nucleoside modified mRNA sequence uridine residues are replaced with a modified nucleoside selected from pyridin-4-one ribonucleoside, 5-aza-uridine, 2-thio-5-azauridine,2-thiouridine, 4-thio-pseudouridine, 2-thio-pseudouridine, 5-hydroxyuridine, 3-methyluridine, 5- carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thiouridine,5-methyl-uridine, 1-methyl-pseudouridine, 4-thio-1-methyl-pseudouridine, 2-thio-1-methylpseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, 4-methoxy-2-thio-pseudouridine, preferably with 1-methyl-pseudouridine

According to another embodiment, preferably in order to further stabilize the mRNA, the coding region of the mRNA according the invention contain an increased G/C compared to the G/C content present in the corresponding coding region for the wild type SARS-CoV-2 spike protein. This is obtained by introducing into the sequence synonymous codon substitutions that allows an increase of G/C ratio.

According to another embodiment, preferably in the mRNA according to the present invention codon usage has been optimized for expression in human cells.

A further object of the present invention is a DNA sequence encoding for the mutated SARS- CoV-2 spike protein, a variant or a peptide thereof as described above.

According to a preferred embodiment, said DNA is contained in a plasmid. Preferably, said plasmid contains at least a promoter and a stop codon.

According to an alternative preferred embodiment, said DNA is contained in a viral vector. Said viral vector may be non-replicating or replicating. Said viral vector is preferably selected from adeno or pox viruses. More preferably, said viral vector is selected from an adenovirus type 5 (Ad5) vector, adenovirus type 26 (Ad26) vector, replication-deficient simian adenovirus ChAdOx1 vector.

The proteins, mRNA or DNA according to the present invention can be prepared by methods well known in the art.

A further object of the present invention is a mutated SARS-CoV-2 spike protein, a variant or peptide thereof or an mRNA or DNA encoding them, as above described, for use in the prevention of COVID-19 in a subject.

A further object of the present invention is a vaccine composition containing a mutated SARS-CoV-2 spike protein, a variant or fragment thereof or an mRNA or DNA encoding them, as above defined.

The vaccine composition is preferably for use in prevention of COVID-19.

The vaccine can be formulated in accordance with well-known techniques for the preparation of protein or nucleic acids based vaccines, as for example described in Batty et al, AdvDrug Delivery Rev 2021, 169: 168-189).

Preferably, said vaccine contains an adjuvant agent.

An "adjuvant" according to the invention is a component that is able to enhance the immunostimulatory properties of the composition.

Said adjuvant is selected from adjuvants well known to the skilled person and suitable for the specific antigen and formulation used.

The vaccine according to the invention can be administered by parenteral administration, preferably intradermal, intramuscular or subcutaneous administration, oral administration or nasal administration.

### EXPERIMENTAL PART

### Example 1

### Bioinformatic analysis

### Materials and Methods

### 1. Interactome analysis

The STRING database [Szklarczyk D et al., Nucleic Acids Res. 2021, 49: D605-D612, doi: 10.1093/nar/gkaa1074], that integrate all known and predicted associations between proteins, including both physical interactions as well as functional associations was used to analyse functional associations between biomolecules. Each protein-protein interaction is annotated with a 'score'. This score does not indicate the strength or the specificity of the interaction, but only the confidence. All scores rank from 0 to 1, with 1 being the highest possible confidence.

### 2. 3D Model selection

Spike 3D model was built based on PDB 6VYB returned to its wild-type form and fully glycosylated. An asymmetric glycosylation of the three protomers was derived by glycoanalyitic data for the N-glycans and O-glycans as published (Casalini L et al, ACS Cent. Sci. 2020, 6:1722-1734, https://doi.org/10.1021/acscentsci.0c01056). The proteins were modeled using Amber14SB force field (Maier J A et al, J. Chem. Theory Comput. 2015, 11:3696-3713, https://doi.org/10.1021/acs.jctc.5b00255) and the carbohydrate moieties by the GLYCAM06j-1 version of GLYCAM06 force field (Kirschner KN et al, J. Comput. Chem. 2008, 29:622-655, https://doi.org/10.1002/jcc.20820). The so prepared structure was used as starting point for Molecular Docking simulations. Topology files were generated with the pdb2gmx GROMACS tool, using the amber99sb forcefield (Lindorff-Larsen K et al, Proteins 2010,78:1950-1958, https://dx.doi.org/10.1002/prot.22711). Protein was inserted in a triclinic box, extending up to 15 Å from the solute, and immersed in TIP3P water molecules (Jorgensen WL et al, J Chem Phys 1983, 79:926-935, https://dx.doi.org/10.1063/1.445869). Counter ions were added to neutralize the overall charge with the genion GROMACS tool. After energy minimizations, the system was relaxed for 5 ns by applying positional restraints of 1000 kJ mol-1 nm-2 to the protein atoms. Following this step, unrestrained MD simulation was carried out for a length of 1 microsecond, with a time step of 2 fs, using GROMACS 2018.3 simulation package (supercomputer Galileo and Marconi-100, CINECA, Bologna, Italy) (Abraham MJ et al., SoftwareX 2015, 1:19-25, https://dx.doi.org/10.1016/j.softx.2015.06.001). V-rescale temperature coupling was employed to keep the temperature constant at 300 K (Bussi G et al, J Chem Phys, 2007 126:014101. https://dx.doi.org/10.1063/1.2408420). The Particle-Mesh Ewald method was used for the treatment of the long-range electrostatic interactions (Darden T et al., J. Chem. Phys 1993, 98:10089, https://doi.org/10.1063/1.464397). The first 5 ns of each trajectory were excluded from the analysis. The trajectory obtained after 1 microsecond MD simulation was clustered in order to obtain representative structures. In particular, the structure used for the docking studies is the first centroid of the first cluster extracted from the MD experiment.

For the estrogen receptor, the XRAY PDB model with code 3OLL was used, containing Estradiol and Nuclear receptor coactivator 1 (Möcklinghoff S et al, ChemBioChem 2010, 11:2251-2254, https://doi.org/10.1002/cbic.201000532).

### 3. Protein-Protein docking procedure

The input of two individual proteins, one for the receptor and the other for the ligand, were provided. In particular, the Spike protein and ER were used as receptor and ligand, respectively. Then, the HDOCK tool performs docking to sample putative binding modes through an FFT-based search method, and then scoring the protein-protein interactions. Finally, the top 100 predicted complex structures were provided, and the best ten hypotheses were visually inspected to confirm the reliability of the calculation. The entire workflow is described (Yan Y et al, Nat Protoc 2020, 15:1829-1852, https://doi.org/10.1038/s41596-020-0312-x).

Given the lack of structural information on the binding mechanism between the Spike protein and the nuclear estrogen receptor (ER), the first check involved the identification of proteins known to interact with estrogen receptors. After identifying these ER interacting proteins, the second step involved the study of any sequence analogies between the spike protein and the ER effector proteins.

### Example 1a

### Nuclear receptor coactivators and LxxLL motif

Many transcription factors and co-factors exhibit a common structural motif that ensures interaction with effector proteins. The motif that participates in these protein-protein interactions is termed LxxLL (LDX), and it is associated with different aspects of transcriptional regulation (Plevin MJ et al, Trends Biochem Sci 2005, 30:66-69. https://dx.doi.org/10.1016/j.tibs.2004.12.001). The LxxLL sequence was originally identified in proteins that bind the activation function-2 (AF-2) region of nuclear receptor ligand-binding domains (LBDs). These motifs are fundamentals in nuclear-receptor regulation with many nuclear-receptor-binding proteins, including co-activators (NCOA-1, 2 and 3) (Heery DM et al, Nature 1997, 387:733-736, https://doi.org/10.1038/42750).

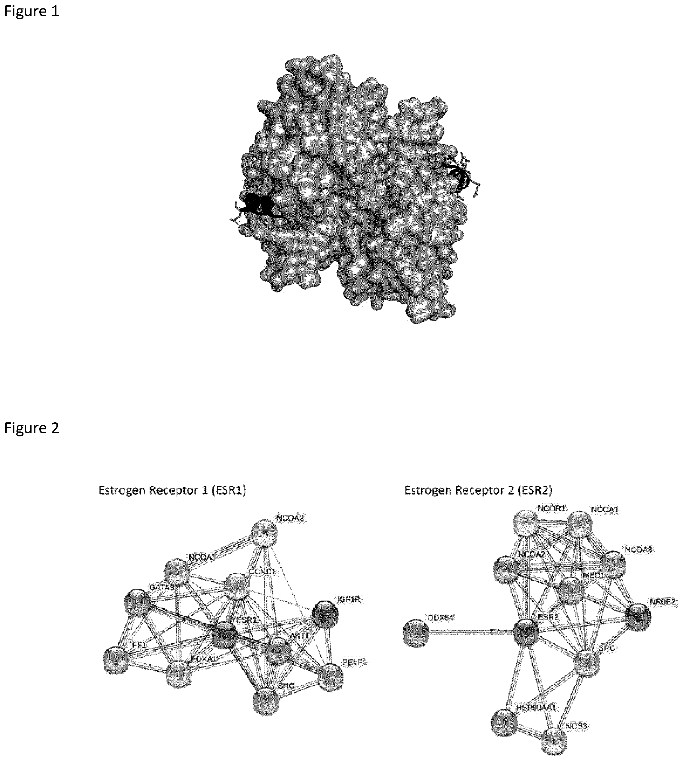

The experimental and structural confirmations which show the interaction between ER and NCOAs, made it possible to focus on the LxxLL motif and starting from this, a mapping of the spike protein sequence was carried out searching for structural motifs and homologous portions of spike able to mimic the interaction between the ER and its nuclear co-activators (Figure 1).

### Example 1b

### Estrogen receptor binding protein network analysis

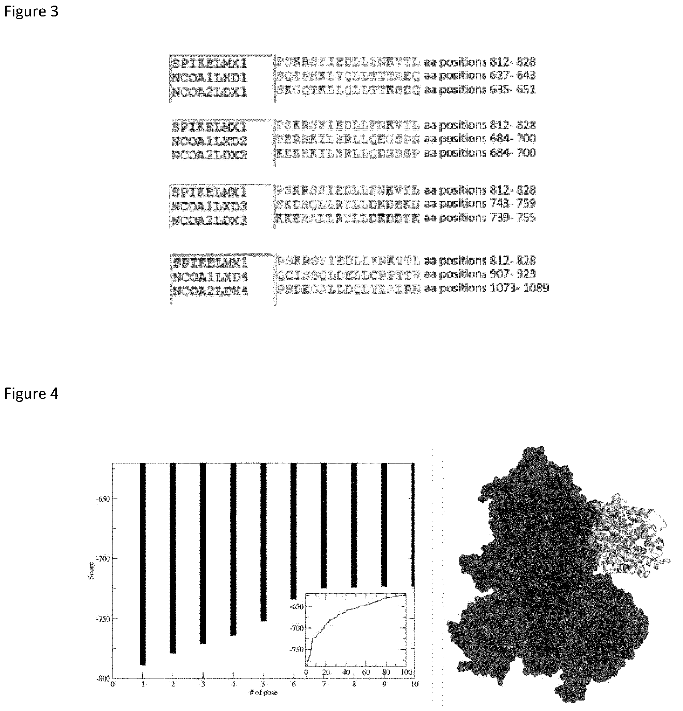

The network of most significant interactions of ER1 and ER2 (both in the red Combining the sequence alignment between NACOs and spike with a three-dimensional analysis of the viral protein, it emerged that the spike protein contains a LDX motif (LPPLL, aa 861-865 in spike wild type) and two motifs homologous to LxxLL (IEDLL, aa 818-822 and LGDIA, aa 841-846, in spike wild type), also valid from a structural point of view (in fact, these regions assumes alpha-helix conformations), in an outer zone which could, in principle, act as interacting region site with ER. Different LDX domain are associated to specific cofactor-receptor interaction and the spike LDX1 resemble NCOA1 LDX4 and belong to Class III domain (Figure 3) (Savkur RS et al, J Pept Res 2004, 63:207-212, https://dx.doi.org/10.1111/j.1399-3011.2004.00126.x

### Example 1c

### Spike protein-ER blind docking

It has been experimentally verified that ER does not bind the Receptor Binding Domain (RBD) of the viral protein (data not shown). To confirm and validate the in silico prediction, the ability of ER to interact in a region other than the viral RBD was evaluated by means of a blind-docking between the two proteins, using the HDOCK server [http://hdock.phys.hust.edu.cn/]. The blind-docking approach does not consider any structural bias and is fully unguided. The best binding hypothesis found puts in evidence a high affinity of ER towards the lateral region of the spike protein, belonging to the so called "fusion peptide portion" (Figure 4).

### Example 1d

### Spike protein-ER motif-oriented docking

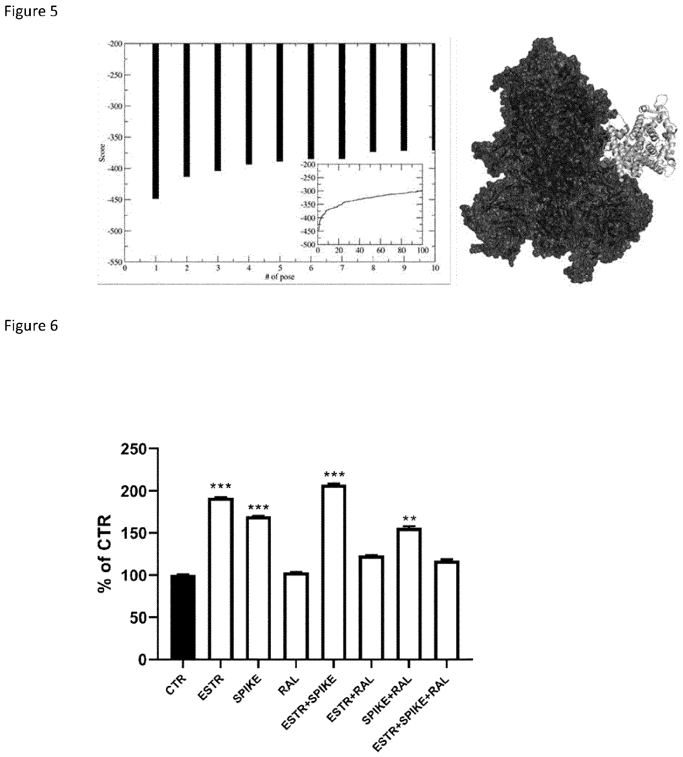

The structura information that ER residues are recognized by NCOAs was used to guide the docking study of ER on spike by optimizing protein-protein interactions. With the protein structures and residue restraints as input, a suitable model was generated by the HDOCK server. Given that the structural information of interaction between ER and NCOA are known, a second docking study was carried out, that considered the ER residues that guarantee interaction with the NCOAs, thus guiding the molecular docking procedure. The best binding hypothesis obtained from the guided docking study is shown in Figure 5 that highlights the binding of the ER to the spike region containing the motifs homologous to the LxxLL pattern.

### Example 2

### In vitro evaluation of proliferation activity in cancer cell lines

### Materials and methods

### Peptides 2, 5 and 7

Starting from homologous and LxxLL-like cores, three hree peptides, named 2, 5 and 7, having the following aminoacid sequences were synthetized:
Peptide 2: SKRSFIEDLLFNKVTL (SEQ ID NO: 16)
Peptide 5: IKQYGDCLGDIAARDLI (SEQ ID NO: 17)
Peptide 7: NGLTVLPPLLTDEMI (SEQ ID NO: 18)

Each of the peptides contains one of the LxxLL motif found in the wild type spike protein. The peptides were synthesized by CASLO ApS (Technical University of Denmark, DTU-Science Park Diplomvej 381, DK-2800 Kongens Lyngby, Denmark), by using a Fmoc-based solid-phase peptide synthesis protocol. Each peptide was acetylated at N-terminus and amidated at C-terminus; they were stored as lyophilized hydrochloride salts. The purity of the peptides was assessed by analytical RP-HPLC (C18-250 mm × 4.6 mm I.D., flow rate of 1 mL/min; absorbance detected at 220 nm) and verified also by MALDI-TOF mass spectrometry.

### Testing concentrations

Spike protein was used at the final concentration of 10 ng/ml in medium. Raloxifene was used at 2 µM final concentration in medium. Estradiol stock solution was prepared in ethanol and used at the final concentration of 1 nM diluted in medium. Peptides were diluted in PBS at 4mg/ml concentration and then 10ug/ml was the concentration used for the assay (final concentration in medium).

### Proliferation Assay

To evaluate cell proliferation, BrdU assay was performed (Abcam, UK). BrdU is incorporated into newly synthesized DNA of actively proliferating cells. 20 µL of the diluted 1X BrdU label will be added to the appropriate wells and incubated 24 hours. BrdU was incorporated into the DNA of dividing cells. To enable antibody binding to the incorporated BrdU cells must be fixed, permeabilized, and the DNA denatured. This is all done in one step by treatment with Fixing Solution. Detector anti-BrdU monoclonal antibody is pipetted into the wells and allowed to incubate for one hour, during which time it binds to any incorporated BrdU. Different washes were performed to wash away the unbound antibody, and horseradish peroxidase-conjugated goat anti-mouse antibody was added, which binds to the Detector Antibody. Then, TMB Peroxidase substrate was added and incubated for 30 minutes at room temperature in the dark. Finally, to stop the reaction, the Stop solution was added. The plate was read using a spectrophotometric microtiter plate reader set at 450 nm

### Breast cancer cell line

MCF-7 cell line is an invasive ductal/breast carcinoma cell hormone-dependent (both estrogen and progesterone receptor-positive).

MCF-7 was obtained from ATCC and grown in DMEM (Dulbecco's Modified Eagle Medium), supplemented with 10% fetal bovine serum (FBS, Corning, USA), penicillin/streptomycin and glutamine (Sigma, USA) at 37 °C in a 5% CO2 and 95% humidified incubator (Thermo, USA). Before treatments, to reduce estrogen levels in FBS the cells were cultured for 24h in phenol red-free medium containing 5% dextran-coated charcoal treated serum, followed by incubation in phenol-red free, serum-free medium, supplemented with 0.2% BSA for at least 24h.

In a first set of experiments, cells were treated with estradiol, wild type spike protein, raloxifene, estradiol and raloxifene, wild type spike protein and raloxifene, estradiol, spike protein and raloxifene for 24 hours and proliferation was measured.

The results are shown in Figure 6. As can be seen both estradiol and spike were able to substantially increase the proliferation of cells and this activity was counteracted by co-treatment with raloxifene. These data demonstrate that wild type spike is able to induce proliferation of breast cancer cells with an ER mediated mechanism.

In a second set of experiments, cells treated with peptides 2, 5 and 7, wild type spike, spike plus raloxifene or raloxifene alone for 24 hours and proliferation was measured.

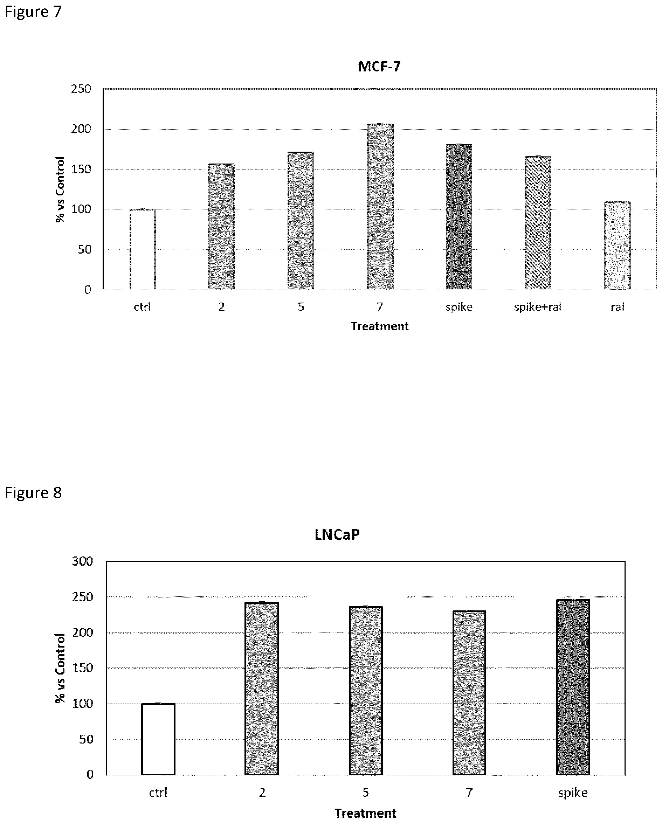

The results are shown in Figure 7. The mean and SD are shown in Table 1 below:

**Table 1**

| **MCF-7** | | | | | | | |
|---|---|---|---|---|---|---|---|
| | **ctrl** | **2** | **5** | **7** | **spike** | **spike+ral** | **ral** |
| **Mean** | 100,0 | 155,7 | 170,6 | 205,5 | 179,9 | 165,3 | 109,5 |
| **SD** | 0,53 | 1,31 | 1,54 | 0,97 | 0,57 | 2,13 | 0,68 |

As can be seen, the peptides are all able to induce proliferation similar to that induced by spike. The data obtained demonstrated that each of the LxxL motifs in wild type spike protein are able to induce activation of the estrogen receptor.

### Prostate cancer cell line

Established androgen-dependent cell line LNCap was used as a model for this study. Cells were cultured in DMEM, supplemented with 10% fetal bovine serum (FBS, Corning, USA), penicillin/streptomycin and glutamine (Sigma, USA) at 37 °C in a 5% CO2 and 95% humidified incubator (Thermo, USA). Before treatments, to reduce estrogen levels in FBS, the cells were cultured for 24h in phenol red-free medium containing 5% dextran-coated charcoal treated serum, followed by incubation in phenol-red free, serum-free medium, supplemented with 0.2% BSA for at least 24h. Then cells were treated with peptides 2, 5 and 7 or wild type spike for 24h and proliferation was measured.

The results are shown in Figure 8. The mean and SD are shown in Table 2 below:

**Table 2**

| **LNCaP** | | | | | |
|---|---|---|---|---|---|
| | **ctrl** | **2** | **5** | **7** | **spike** |
| **Mean** | 100,0 | 242,2 | 236,2 | 230,2 | 245,20 |
| **SD** | 0,21 | 0,72 | 0,24 | 0,7 | 0,29 |

The results obtained demonstrate that both the peptides and spike protein are able to induce proliferation, by activation of the androgen receptor.

### Example 3

### Proteomic analysis

Human monocytes THP-1 were purchased from ATCC and cultured following manufacturer's instructions. Then THP-1 were differentiated using 20 ng/ml phorbol 12-myristate 13-acetate (PMA) for 72 hours, followed by LPS stimulation (100 ng/ml) for 24 hours. Then, cells were treated with estradiol, raloxifene and wild type spike, wild type pike + raloxifene, estradiol + wild type spike, estradiol + raloxifene + wild type spike, at the concentrations used in Example 2, for 24h, the media were collected to assess the proteome profile assay was carried out.

In details, conditioned media from THP-1 treated cells were centrifuged to remove particulates and assayed immediately. The sample amount was adjusted as suggested (500 µl). The reagents will be prepared following manufacturer's protocols (R&D, USA). Briefly, the membranes were incubated with Array Buffer 6 for 1 hour on a rocking platform shaker. The samples were prepared by adding 1 ml of Array Buffer 4 in two separated tubes and then 15 µl of reconstituted mouse cytokine detection antibody cocktail (> 40 mouse cytokines) to each prepared sample and incubated 1 hour. Then, the Array Buffer 6 was aspirated and replaced with the sample/antibody mixtures and incubated at 4° C overnight. The following day, membranes were washed 3 times, and Streptavidin-HRP (1:2000) was incubated for 30 minutes at room temperature on a rocking platform shaker. The membranes were washed again, and 1 ml of prepared Chemi Reagent mix was placed onto each membrane. Multiple exposure times were acquired using UVITEC digital analyzer (Alliance, Cambridge, UK). The positive signals seen in the developed membranes can be identified by placing the transparency overlay template on the array image and aligning it with pairs of reference spots in the three corners of each array. Reference spots are included to demonstrate that the array has been incubated with Streptavidin-HRP during the assay procedure. Pixel densities (average signals of pair of duplicates which represent each cytokine) were analyzed by Fiji software, and the average background was subtracted from each spot.

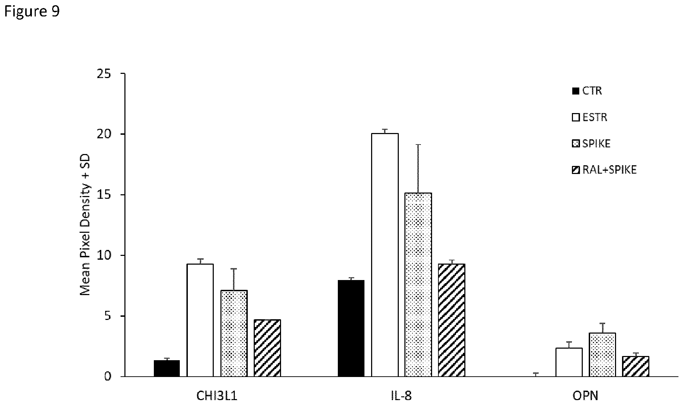

The results are reported in Figure 9. As can be seen, IL-8 appears the most abundant, being up-regulated by both estradiol and wild type spike but especially by their combination. Raloxifene was effective in counteracting the effect of spike, alone and with estradiol. The non-enzymatic chitinase-3 like-protein-1 (CHI3L1) is up-regulated by both estradiol and wild type spike but raloxifene was able to counteract only the effects of spike alone or in combination with estradiol.

## Claims

1. A mutated SARSCoV-2 spike protein according to the invention having the aminoacid sequence of SEQ ID NO: 2.

2. A variant of the mutated SARSCoV-2 spike protein claimed in claim 1.

3. A variant as claimed in claim 2, wherein at least two of the aminoacids alanine at positions 892, 899 and 942 of SEQ ID NO: 2, phenylalanine at position 817 of SEQ ID NO: 2, lysine at position 986 of SEQ ID NO: 2 and valine at position 987 of SEQ ID NO: 2 are replaced by proline.

4. A variant as claimed in claims 2 or 3, wherein the aminoacids arginine at positions 682, 683 and 685 of SEQ ID NO: 2 are replaced by glutamine.

5. An immunogenic fragment of the mutated SARSCoV-2 spike protein as claimed in claim 1, comprising at least one sequence selected from the following aminoacid sequences:
- aminoacid sequence from position 818 to position 822 of SEQ ID NO: 2,
- aminoacid sequence from position 841 to position 845 of SEQ ID NO: 2, and
- aminoacid sequence from position 861 to position 865 of SEQ ID NO: 2.

6. An immunogenic fragment as claimed in claim 5, comprising a sequence corresponding the aminoacid sequences of from aminoacid 818 to aminoacid 865 of SEQ ID NO: 2.

7. An immunogenic fragment as claimed in claim 5 or 6, further comprising the aminoacid sequence from position 865 to position 1209 of SEQ ID NO: 2.

8. An immunogenic fragment as claimed in claim 5 to 7, further comprising the aminoacid sequence from position 471 to position 817 of SEQ ID NO: 2.

9. An immunogenic fragment as claimed in claims 5 to 8, further comprising the aminoacid sequence from position 232 to position 470 of SEQ ID NO: 2.

10. A protein comprising the sequence of a mutated SARSCoV-2 spike protein, variant or immunogenic fragment as claimed in claims 1 to 9, and one or more additional sequences.

11. An mRNA encoding for a mutated SARS- CoV-2 spike protein, a variant, a peptide or protein as claimed in claims 1 to 10.

12. An mRNA as claimed in claim 11, having a sequence wherein uridine residues are replaced with a modified nucleoside selected from pyridin-4-one ribonucleoside, 5-aza-uridine, 2-thio-5-azauridine,2-thiouridine, 4-thio-pseudouridine, 2-thio-pseudouridine, 5-hydroxyuridine, 3-methyluridine, 5- carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thiouridine,5-methyl-uridine, 1-methyl-pseudouridine, 4-thio-1-methyl-pseudouridine, 2-thio-1-methylpseudouridine, 1-methyl-1-deaza-pseudouridine, 2-thio-1-methyl-1-deazapseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, 4-methoxy-2-thio-pseudouridine, preferably with 1-methyl-pseudouridine.

13. A DNA encoding for a mutated SARS- CoV-2 spike protein, variant, peptide or protein as claimed in claims 1 to 10.

14. A DNA as claimed in claim 13, which is contained in a plasmid.

15. A DNA as claimed in claim 13, which is contained in a viral vector, preferably selected from adeno or pox viruses.

16. A mutated SARS-CoV-2 spike protein, variant, immunogenic fragment, mRNA or DNA as claimed in claims 1 to 15, for use in the prevention of COVID-19 in a subject.

17. A vaccine composition containing a mutated SARS-CoV-2 spike protein, variant, immunogenic fragment, mRNA or DNA as claimed in claims 1 to 14.
